# EUROPEAN PATENT APPLICATION

(11) **EP 1 589 097 A1**
(43) Date of publication of application: **26.10.2005**
(21) Application number: 04291067.9
(22) Date of filing: 23.04.2004
(51) Int. Cl.: C12N 5/06, C12N 5/08, C12N 5/02, G01N 33/50

(54) **Isolation and culture of a high purity population of cone photoreceptor cells, and biological applications thereof**

(71) Applicant: INSERM, 75654 Paris Cédex 13 (FR)
(72) Inventor: Picaud, Serge, 77210 Avon (FR); Balse, Elise, 75013 Paris (FR); Forster, Valérie, 75012 Paris (FR); Fuchs, Céline, 75013 Paris (FR); Teissier, Luc-Henri, 67000 Strasbourg (FR); Sahel, José-Alain, 75013 Paris (FR)
(74) Representative: Paris, Fabienne

(57) **Abstract**

The present invention provides a process and kit for isolating cone photoreceptor cells from retinal tissue with a purity level of at least 80%, typically of about 90%. The isolation process uses a PNA-panning procedure conducted on dissociated retinal tissue.

The present invention also provides a culture medium enabling the in vitro survival and development of such isolated cone cells.

The means of the invention are applicable to adult mammalian cone cells, and more particularly to adult human cone cells. They have the advantage of being applicable to pathologic or otherwise altered cone cells, and thus give access to the screening of compounds capable of showing neuroprotective activity on adult cone cells.

## Description

### FIELD OF THE INVENTION

The present invention generally relates to the field of cone photoreceptor cell production by isolation from a retinal tissue, and to the biological applications thereof, notably as screening tools to identify compounds capable of showing anti-degenerative properties on cone photoreceptors.

### BACKGROUND OF THE INVENTION

Photoreceptor degeneration is the cause of blindness in diseases such as retinitis pigmentosa and age macular degeneration. In these diseases, the cause of blindess or low vision is due to cone photoreceptor degeneration. Night blind people can lead a normal life especially in industrial countries and useful vision can still prevail despite a 95% loss of cones (Geller and Sieving, 1993). Preventing cone cell loss thus appears as a main target in therapeutic strategies. In retinitis pigmentosa, although most causative mutations affect rod photoreceptor specific genes, rod apoptosis is followed by a secondary cone degeneration. This secondary cone photoreceptor degeneration was attributed to a rod-dependent trophic factor required for cone survival (Mohand-said *et al.,* 1998 ; Fintz *et al.,* 2003).

All these results and considerations urged the need to screen cone photoreceptor survival factors, however this screening was limited by the lack of a pure photoreceptor cell culture.

There have already been some attempts in this respect, but they were either limited to non-adult cells (*e.g*. embryo retinal cells), and/or non applicable to mammalian cells, and/or did not achieve a sufficiently pure degree of cone isolation.

For example, cell suspensions from 8-d chick embryo retina have been cultured *in vitro* for up to 7 days in low-density glial-free monolayers, and thereby led to a partial differentiation into developing chick cones (Adler *et al.,* 1984 ; Adler, 1990 ; Fintz *et al.,* 2003). The chick embryo retinal cell cultures obtained therefrom could reach a purity of up to 70%. However such cultures may not be representative of the physiology and biochemistry of an adult cone cell. They also cannot provide information about diseased adult cone cells.

Cell cultures containing adult mammalian retinal cells have also been produced, but they included both cone and rod photoreceptors (Hicks *et al.,* 1994 ; Gaudin *et al.,* 1997 ; Picaud *et al.,* 1998a). When the retina was sectioned in its thickness to isolate the photoreceptor layer, rods and cones survived only on a glial feeder cell layer (Picaud *et al.,* 1998b). This approach therefore did not allow to separate rods from cones. It however provided evidence for a strict photoreceptor dependence on glial cells. Nevertheless the rod dependence for cone survival could not be addressed on this model.

Another method of progressive dissociation has been described that provided relatively pure photoreceptor cell culture, but still in a rod-cone ratio of 2:3.1 (Traverso *et al.,* 2003). This preparation was used to confirm that trophic factors like EGF and FGF2 can postpone photoreceptor degeneration.

To the Applicant's knowledge, there has not been a description of means that would enable the isolation of a substantially pure population of adult cone photoreceptor cells. Particularly needed would be isolation means that would not promote the selection of a particular cone sub-population (S-cones *vs.* M/L-cones), *i.e*. that would introduce no selection bias, thereby giving a reliable *in vitro* model of the cone cell distribution in the tissue from which it derives. Particularly needed would also be isolation means that would be applicable to mammalian cone cells, and more particularly human cone cells. Most particularly needed would be isolation means that would be applicable to diseased adult cone cells.
Further to such isolation means, there also is a need for culture means that would enable to easily and reliably maintain isolated cone cells in *in vitro* culture.

### SUMMARY OF THE INVENTION

The present invention provides a process and kit for the production of a cone photoreceptor population of high purity. It thereby provides a high purity population of cone photoreceptor cells obtainable therefrom, and with means enabling the easy and reliable conducting of an *in vitro* culture of cone photoreceptor cells. The present invention also relates to the biological applications of such cone photoreceptor cell population and culture.

It more particularly relates to means enabling the isolation and culture of a cone cell population of high purity, wherein said means advantageously do not select a particular cone sub-population, thereby giving access to a cone culture that is reliably representative of the cone cell distribution that was initially present in the retinal tissue from which the cones have been isolated.
They also have the advantage of being applicable to, and efficient for adult cone photoreceptors. They further are applicable to mammalian cone cells, and more particularly to human cone cells.
The invention thereby gives access to genomics and proteomics, as well as to means for screening for compounds that have therapeutic and/or palliative and/or anti-degenerative effects on pathological or otherwise altered cone cells.

In the present application, reference is made to various scientific publications. These publications are listed in the "BIBLIOGRAPHIC REFERENCES" section which is located before the "CLAIMS" section.

### DETAILED DESCRIPTION

In order to characterize cone photoreceptors and screen trophic factor supporting their survival, the present invention provides a process that notably allows the specific isolation and culture of viable adult cone photoreceptors by "lectin-panning" using the peanut germ agglutinin lectin (PNA).

PNA specifically interacts with the cone domains of the interphotoreceptor matrix in the retinal tissue (Kivela and Tarkkanen, 1987; Uehara *et al.,* 1997; Yan *et al.,* 1995; Ahnelt and Kolt, 2000).
But, PNA was also reported to bind differently to the different populations of cone photoreceptors (Ebrey and Koutalos, 2001).

The present invention demonstrates that PNA can nevertheless be successfully used to mediate isolation of cone photoreceptor cells from an adult mammalian retinal tissue, and that PNA binding can still be operative and selective even though the tissue to which it is applied has undergone extracellular matrix dissociation.

It further shows that PNA can mediate said isolation without introducing a selection bias in the cone sub-populations, *i.e*. without promoting the adherence of a particular cone sub-population to the detriment of another particular cone sub-population.
It is indeed herein shown that PNA lectin-panning can select both S- and L/M-cone photoreceptors, and is efficient even though the retinal cells have undergone extracellular matrix lysis for cell dissociation.

The isolation means of the invention have the further advantages of being applicable to:
- adult mammalian cone cells, and more particularly human cone cells, and to
- healthy as well as pathological or otherwise altered cone cells.

The present invention further provides means enabling the *in vitro* culture of such isolated cone cells, demonstrating that retinal Müller glial cells can release diffusible factors that promote both adult cone photoreceptor survival and development *in vitro.*

The isolation and culture means of the invention thus give access to previously unmet industrial needs, notably for screening for compounds capable of showing therapeutic and/or palliative and/or anti-degenerative effects on adult diseased cone cells.

The present invention thus relates to a process for the *in vitro* production of a population of cone photoreceptors by isolation from a retinal tissue with a cone purity of at least 80%, typically of about 90%, said process promoting no selection of the S-cone sub-population *vs.* the L/M-cone sub-population.

Said process comprises:
- optionally, chopping the retinal tissue into small fragments, preferably under conditions non deleterious to retinal cell biology, *i.e*. under conditions that do not substantially impair the growth potential of the retinal cells, for example in cold CO2-independent medium,
- dissociating the extracellular matrix of the retinal tissue or retinal tissue fragments enzymatically and/or mechanically and/or chemically, so as to dissociate the retinal cells from each other without substantially altering their cellular integrity, *i.e*. without lysing the retinal cells,
- optionally, suspending the dissociated cells in a solution,
- placing said dissociated cells in contact with PNA (peanut germ agglutinin) under conditions enabling cell-PNA binding,
- recovering the PNA-bound cells, and optionally *in vitro* culturing the PNA-bound cells,
whereby said population of photoreceptor cones is obtained.

Said PNA is a commercially available and widely distributed product; for example, it is available from Sigma (reference : L0881)

Said purity percentage is assessed by counting the number of cells that are arrestin-positive, and relating it to the total number of cells that are observed under transmitted light.
To detect arrestin-positive cells, the skilled person can use the RT-PCR technique to detect cone arrestin mRNA (*cf.* example below, section *"Material and Methods",* sub-section *"single-cell RT-PCR").* Alternatively, an anti-arrestin antibody selective for cone arrestin protein can be used, *e.g.* an antibody to the QKAVEAEGDEGS epitope (*cf.* example below, section *"Material and Methods",* sub-section *"Histology"*)*.*
As illustrated in the Example below, a purity percentage of 92.57 ± 2.12 % assessed by anti-arrestin antibody (human anti-cone arrestin antibody; hCAR) was evaluated to be 92.06 ± 4.82 % by RT-PCR on arrestin mRNA.

The cell population obtained by the present invention comprises a majority of cone cells (namely at least 80%). There may nevertheless remain some "contaminant" cells, which originate from the retinal tissue on which the cell isolation has been performed, but which are not cone cells. These "contaminant" cells typically are:
- rod bipolar cells, which typically may account for 4 to 6% of DAPI-stained nuclei, and also
- very few glial cells, which were identified by their large DAPI-stained cell bodies.

These observations indicated that PNA-panning give rise to a high degree of purification of cone photoreceptors in culture.

Advantageously, notably compared to prior art techniques, as well as in the perspective of the possible uses that it offers, said retinal tissue can be an adult tissue.
It may be a mammalian retinal tissue (primate or non-primate retinal tissue), *e.g*. a human retinal tissue, or an animal but non-human retinal tissue such as pig, rat, mouse retinal tissue.

Said retinal tissue can be a healthy tissue, as well as and advantageously a pathologic tissue, *e.g*. a tissue afflicted by a photoreceptor degeneration (cone and/or rod dysfunction).
In the process of the invention, said retinal tissue can therefore be a pathologic retinal tissue that undergoes or has undergone photoreceptor degeneration. This is a particularly advantageous aspect of the invention, as it enables methods for screening for compounds capable of showing a protective and/or anti-degenerative effect on cone cells, as well as genomics and proteomics applications, *e.g.* identifying genes and proteins that are specific or characteristic of the cone cells compared to the other retinal cells, or genes and proteins that are specific or characteristic of pathological cone cells compared to healthy cone cells (and vice versa).

Said pathologic retinal tissue can originate from an individual afflicted with an inherited or acquired disease involving photoreceptor degeneration, such as retinitis pigmentosa and age macular degeneration, or other maculopathies.
Said individual can advantageously be a mammal, such as a human or a non-human animal (*e.g*. pig, rat, mouse).

Dissociation of extracellular matrix of the retinal tissue or retinal tissue fragments can be achieved by any means available to the skilled person, as long as it allows for retinal cell dissociation without leading to cone cell lysis.

An enzyme, such as a proteolytic enzyme, can advantageously be used to that effect. Examples of such proteolytic enzymes notably include papain and trypsin. Papain is *e.g*. available from Worthington, and trypsin from Sigma.

The skilled person can adjust the dissociation conditions, such as temperature, enzyme concentration, and dissociation duration, as a function of the enzyme used and of the retinal tissue to which it is applied. Illustrative conditions are mentioned in the Example below. The skilled person may also use an enzyme activator, such as cysteine, if desired.

Dissociation can alternatively or additionally be performed mechanically and/or chemically. According to a particular aspect of the invention, dissociation is performed enzymatically and mechanically (see *e.g.* Example below, *"Material and Methods"* section, *"Cell cultures"* sub-section).

Before placing said dissociated cells in contact with PNA, the process of the invention can additionally comprise the step of saturating those sites which may introduce an aspecific lectin binding. Such a saturation may be achieved by any appropriate means available to the skilled person, *e.g.* by placing the dissociated cells obtained by disruption of said retinal tissue in contact with BSA.

According to an advantageous aspect of the invention, said PNA can be bound to a solid support, *e.g.* via anti-PNA antibody, such as the anti-PNA IgG available from Sigma under reference A4404.
Any solid support appropriate to cell binding can be used. It can *e.g.* be a cell culture plate, a glass coverslip, magnetic beads coupled with antibody anti-PNA.

Said placing in contact of the dissociated retinal cells with PNA can be achieved by any appropriate means that are appropriate to the binding of a lectin to a cell, such as by panning, MACS (magnetic activated cell sorting), or FACS (fluorescent activated cell sorting technique).

Once a cone population of a purity in cone cells as high as at least 80% has been produced, there comes the problem of maintaining such a population alive for a sufficiently long period of time, *e.g.* more than a week, such that they can be used in applications wherein cells shall preferably be living, such as screening protocols.
The present invention addresses this problem, and thereby discloses a fully comprehensive method for producing cone photoreceptors *in vitro.* It thus enables to perform automated screening procedures for *e.g.* the identification of therapeutically useful compounds.
A further and complementary aspect of the invention thus lies in the culture of the isolated cone photoreceptors.
The present invention notably demonstrates that Müller glial cells synthesize and extracellularly release trophic factors which promote the survival and development of cone cells.
The present invention hence provides a conditioned medium that is appropriate for *in vitro* cone cultures. The cells used to condition this medium are Müller glial cells.
Müller glial cells are available by *e.g.* purification from retinal suspensions, following the procedure described by Guidry (1996) [see Example below, *"Material and Methods"* section, *"Müller glial cell conditioned medium"* sub-section].
The "pre-conditioned" culture medium is selected as being suitable for cone survival, and may for example be NBA™ (Neurobasal Medium, available from Invitrogen), optionally supplemented by B27 and glutamine (*cf.* sub-section entitled *"Müller glial cell conditioned medium"* in Example Section *"Material and Methods"* below), or a DMEM/F12 medium (available *e.g.* from Invitrogen), or Ames (available *e.g.* from Sigma).
Once they have been purified, the Müller glial cells may first be *in vitro* cultured on a culture medium that is favourable to their own growth, such as *e.g.* DMEM-10%FCS, and then placed on the "pre-conditioned" medium, *e.g.* by substituting the first pro-growth culture medium by the pre-conditioned medium.

The process of the invention hence advantageously comprises *in vitro* culturing the PNA-bound cells on a conditioned culture medium obtainable by collection of the culture medium after *in vitro* culture of Müller glial cells.

The present invention also relates to any population of cone photoreceptors that is obtainable by the process of the invention. Such a population has a purity of at least 80% in cone cells, typically of 85-95%, *e.g.* about or higher than 90%.
As apparent from the description of the production process, said cone cells can advantageously be adult cone cells.
They can be mammalian (primate or non-primate) cone cells, *e.g.* human cone cells, or animal but non-human cone cells such as pig, rat, mouse cone cells.
As above-mentioned, said cone cells can derive from a healthy retinal tissue, as well as and advantageously from a pathologic retinal tissue afflicted by a photoreceptor degeneration (cone and/or rod dysfunction).
Said cone cells can thus be healthy cone cells, as well as and advantageously pathologic cone cells afflicted by a cone photoreceptor degeneration, such as those induced by retinitis pigmentosa, age macular degeneration, or any other maculopathy.
As previously mentioned, the process of the invention has the advantage of introducing no selection bias in the cone isolation: S-cone and L/M-cone photoreceptors are equally isolated.
If the retinal tissue to which the process of the invention is applied is a mammalian tissue, the population of cone photoreceptors will hence comprise both S-cone and L/M-cone photoreceptors.

The present invention also relates to any use of a culture medium obtainable by collection of the culture medium after *in vitro* culture of Müller glial cells for the *in vitro* culture of isolated cone cells.
It more particularly relates to a process for the production of a culture medium that is adapted to the *in vitro* culture of a population of cone photoreceptors, such as the population according to the invention. Said process comprises conditioning a culture medium that is suitable for cone survival with Müller glial cells, *e.g.* by culturing Müller glial cells onto a culture medium that is suitable for cone survival, and collecting the resulting culture medium, this collected culture medium being a conditioned culture medium adapted to the *in vitro* culture of a population of cone photoreceptors, such as a high purity population of cones according to the invention.
The pre-conditioned culture medium that is selected as being favourable to cone survival and that is intended to receive said Müller glial cells for culture is NBA™ culture medium, optionally supplemented by B27 and glutamine, or DMEM/F12, or Ames.

The conditioned medium is typically collected at day 1 or 2 of the Müller glial cell culture on pre-conditioned medium.

The present invention also relates to a cell culture which comprises:
- a population of cone photoreceptors according to the invention, and
- a conditioned culture medium obtainable by the process for the production of a culture medium of the invention.

The present invention also encompasses a multi-well culture plate, comprising in at least one of its wells, a population of cone photoreceptors according to the invention, and optionally a conditioned culture medium obtainable by the process for the production of a culture medium of the invention.

Are also encompassed by the present invention those kits that enable the implementation of the invention.

The present invention therefore encompasses a kit for the *in vitro* production of a population of cone photoreceptors by isolation from a retina tissue with a cone purity of at least 80%, typically of about 90%, said kit comprising:
- a proteolytic enzyme, and
- PNA, optionally bound onto a solid support,
- and optionally, a culture medium intended for the *in vitro* culture of said cone cells, this culture medium being obtainable by collection of the culture medium after *in vitro* culture of Müller glial cells.
The kit may comprise either the culture medium resulting from collection of the culture medium after *in vitro* Müller glial cells (=the conditioned medium), or the Müller glial cells themselves and a (still uncultured yet) culture medium (= the pre-conditioned medium) which is intended to be first seeded by Müller glial cells, and then collected as resulting (conditioned) culture medium.
Said proteolytic enzyme may be any enzyme that allows the dissociation of the extracellular matrix of a retinal tissue or retinal tissue fragments, so as to dissociate the retinal cells from each other without lysing the retinal cells. Examples of such enzymes notably comprise papain, trypsin.

The present invention hence enables the isolation and long-lasting culture of populations of cone photoreceptors that reliably reflect the retinal tissue from which they have been isolated.
It therefore enables to screen for compounds capable of showing a protective and/or anti-degenerative effect on cone photoreceptors.
The present invention hence also relates to a screening method which comprises:
- providing a population of cone photoreceptors that undergoes or has undergone cone degeneration, using the process according to the invention (*i.e*. from a retinal tissue afflicted by a photoreceptor degeneration such as retinitis pigmentosa, age macular degeneration or another maculopathy), said population being optionally seeded in a well of a multi-well plate,
- placing said population of cone photoreceptors in contact with a candidate compound,
- assessing whether said candidate compound induces a protective and/or anti-degenerative effect on said population of cone photoreceptors, *e.g.* by assessing whether it promotes cone cell viability or reduces cone cell mortality.
Candidate compounds include EGF, FGF2, and RDCVF1 and RDCVF2 (Rod-Derived Cone Viability Factor ; see WO 02/081513).
Screening can be achieved *e.g.* by a Live/Dead assay (count of living and dead cells; e.g. using the Live/Dead assay kit from Molecular probes, Eugene, USA), by total cell count (DAPI staining), cone cell count (ELISA), and/or by morphometric determination.
Said population of cone photoreceptors can be a population of animal but non-human animal cone photoreceptors, such as pig, rat or mouse cone photoreceptors.
For validation studies, a population of human cone photoreceptors can advantageously be used.

The present invention also relates to the use of a population of cone cells according to the invention in genomics and/or proteomics analysis processes, *e.g.* to identify a gene/protein that is specific or characteristic of the cones cells compared to the other retinal cells, to identify a gene/protein that is specific or characteristic of a pathological cone cell compared to a healthy cone cell (and vice versa).

The present application hence relates to any use of PNA for the isolation of cone photoreceptors, and to a culture medium enabling the survival of such isolated cones, said culture medium containing trophic factors that are extracellularly released by cultured Müller glial cells.

### BRIEF DESCRIPTION OF THE FIGURES

In the examples below, reference is made to the following figures:

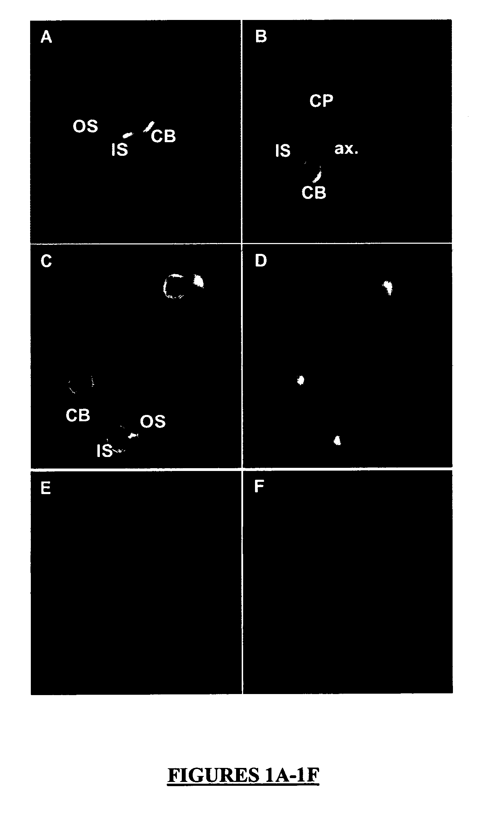

**Figure 1.** Photomicrographs of freshly lectin-panned cells. (A-B) Cone photoreceptor morphology observed under transmitted light with Nomarski optics immediately after the panning procedure. OS: outer segment IS: inner segment, CB: cell body, Ax.: axon and CP: cone pedicle. (C) Immunolabelling with hCAR, an antibody directed against the human cone-arrestin and (D), the nucleus marker DAPI. (E-F) Evidence for the presence of both cone populations in the lectin-panning preparation. Freshly panned cells were immunopositives for either S-opsin (F) or M/L-opsin (F). Opsins are shown in red and the nucleus marker DAPI in blue.

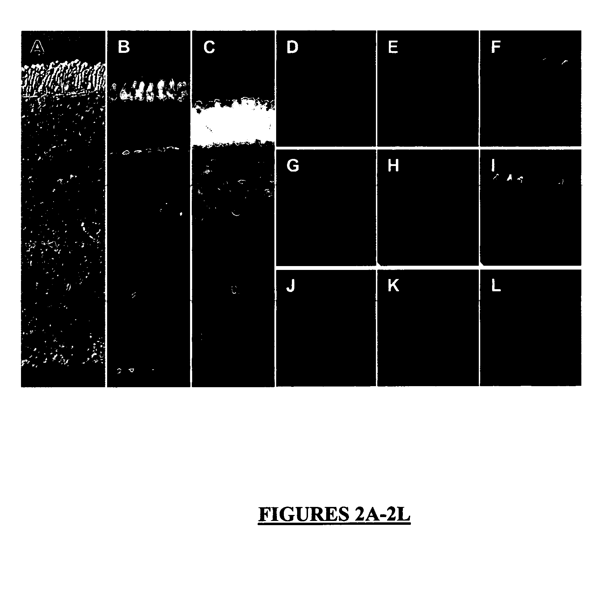

**Figure 2.** Photomicrographs of pig retina immunofluorescence for cone photoreceptors markers. Pig retinal section observed under Nomarski optics (A) or epifluorescence after PNA labeling (B) or DAPI staining of the nuclei (C). (D-L) are focuses of the outer retina after labeling with PNA (D, G, J, green), hCAR (E,), L/M-opsin (H) or S-opsin (K) (red). (F-L) correspond to merged images of PNA, opsins and nucleus stainings. Note for instance that cone photoreceptors stained with PNA are also immunopositives for hCAR or cone-opsins, indicating that PNA-lectin equally labels both S-wavelength and M/L-wavelength cone photoreceptors in the pig retina.

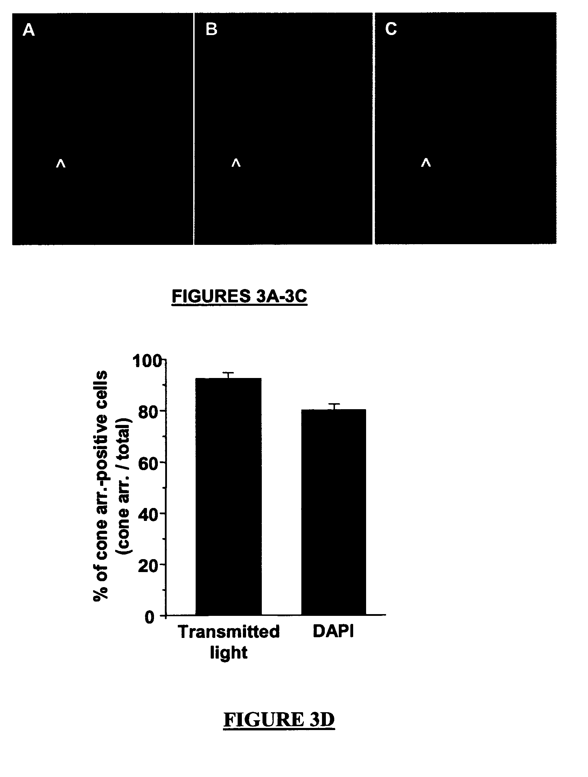

**Figures 3A-3D**. Purity assessment of the lectin-panned cells. After 2 days in culture, isolated cells were counted under transmitted light (Figure 3C) or under epifluorescence illumination with the nucleus marker DAPI (Figure 3A) and the human anti-cone arrestin antibody (hCAR) (Figure 3B). Differential counting revealed a 92.57 ± 2.12 % purity when comparing hCAR and phase contrast while comparison with DAPI is only 80.2 ± 2.32 % (Figure 3D). (*) indicates picnotic cells that are discarded for counting in phase contrast.

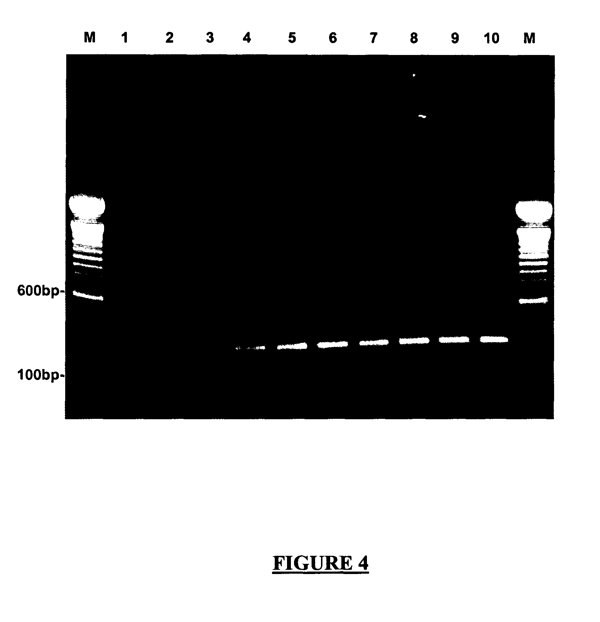

**Figure 4.** Analysis of single-cell RT-PCR. A 100bp DNA standard marker ladder is shown on lanes M. Lanes 1-3 were negative controls: PCR buffers, intra-cellular pipette buffer and extra-cellular buffer, respectively. Lanes 4 to 10 show a 291bp amplified DNA product corresponding to C-arrestin mRNA from a single cell. DNA products were resolved in a 1.2 % agarose gel and visualized with ethidium bromide.
**Figures 5A-5E.**
   **Figure 5E.** Glial influence on cone photoreceptors survival. ANOVA of the number of hCAR-positive cells at different time in culture showed a significant "Day" effect [F(1/12) = 63.62, p < 0.001] that was due to a 64 % decrease in the total number of labeled cone photoreceptors at day 7 compared to day 4 of culture. ANOVA also showed a significant effect of the "Culture Medium" [F(1/12) = 8.94, p < 0.05] that reflected a better survival in RMG CM compared to the control medium at any time in culture. (*) Different from the control condition at the same day (p < 0.001); (**) Different from the control condition at Day 4 (p < 0.01); (#) Different from Day 4 (p < 0.05).
   **Figures 5A-5C.** Glial influence on neuritis outgrowth. (A-B) Control medium and (C-D) Glia-conditioned medium.
**Figures 6A-B.** Voltage-activated conductances in cone photoreceptors recorded either *in situ* or after the lectin-panning-mediated purification.
   **Figure 6B.** *I-V* relationships of cells voltage-clamped at -70 mV and submitted to voltage steps of 10 mV increments, ranging from -120 to +50 mV.

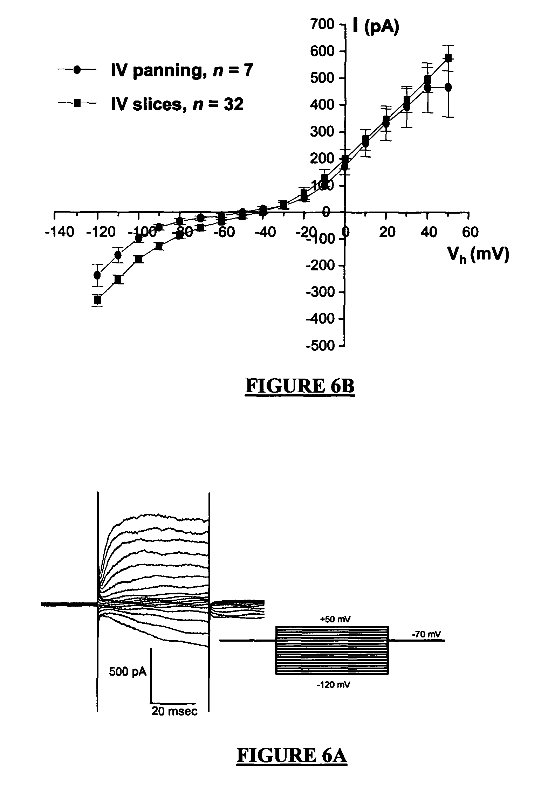

   **Figure 6A.** Sample traces showing two representative cells recorded either *in situ* or *in vitro,* following the dissociation. Note for instance the highly homogenous whole cell conductances of the two conditions.

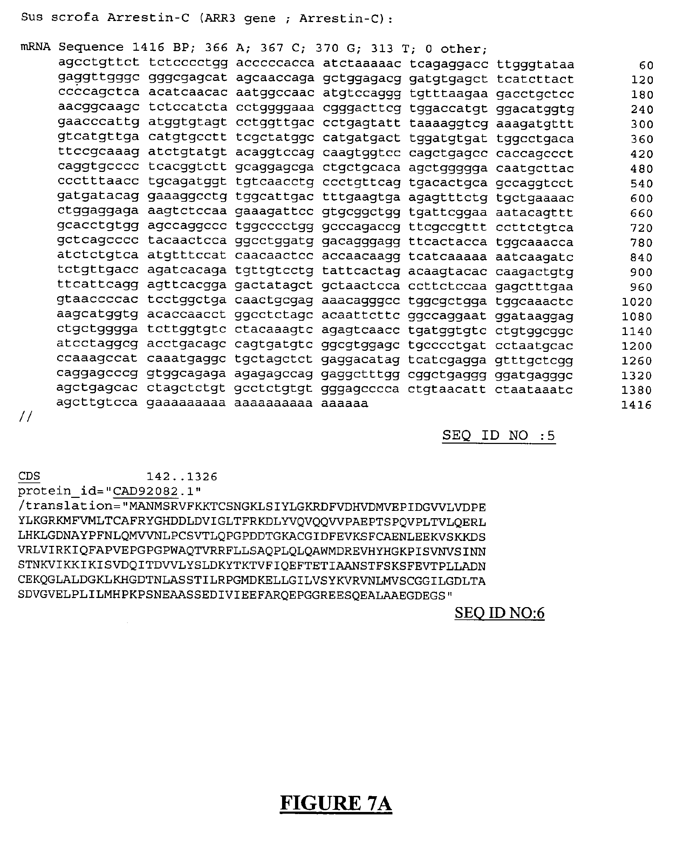

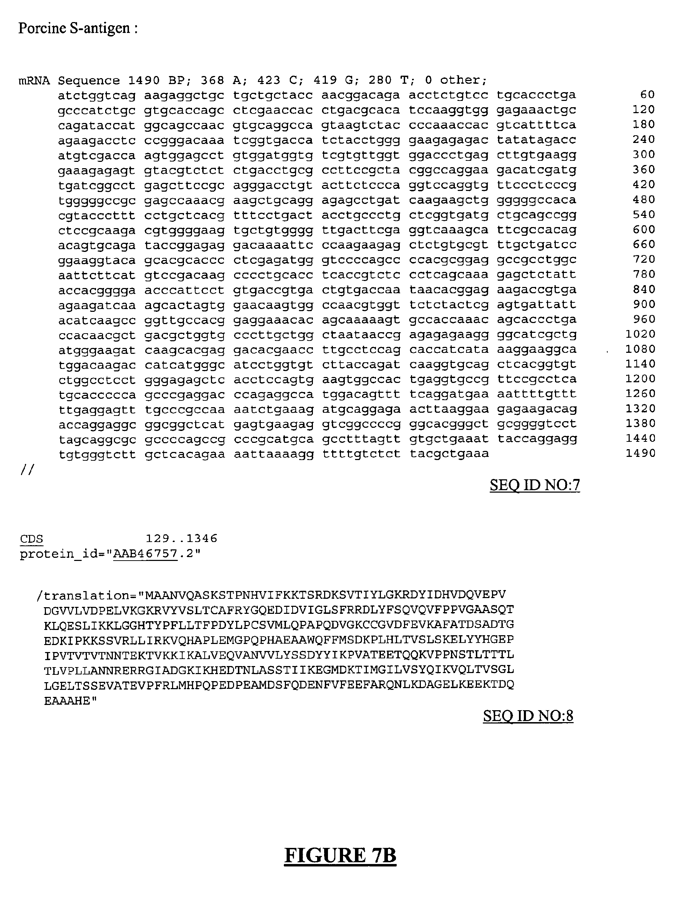

**Figure 7A.** *Sus scrofa* arrestin-C (ARR3 gene) nucleotide sequence (EMBL accession number AJ564496 ; SEQ ID NO:5 ; encoded protein SEQ ID NO:6) ; and **Figure 7B.** *Sus scrofa* S-antigen mRNA (accession number S82664 ; SEQ ID NO:7 ; encoded protein SEQ ID NO:8).

The following examples are offered by way of illustration and are not intended to limit the invention in any manner.

### EXAMPLES:

### Material and Methods

### Cell cultures

Cell suspensions were prepared as described previously (Gaudin *et al.,* 1996, Picaud *et al.,* 1998a). Briefly, adult pig eyes were obtained from the local slaughterhouse. After a short alcohol treatment, the cornea, lens and vitreous humor were removed from the eyecup. The retinal tissue was detached from the pigmented epithelia and the underlying sclera. The retina was chopped into small fragments in cold CO₂-independent medium (Invitrogen). Retinal pieces were incubated for 20 min at 37°C in a papain solution (1 U/µl; Worthington) previously activated by L-cystein (Sigma). The enzymatic reaction was stopped by adding 1ml of neurobasal medium (NBA; Invitrogen) supplemented with 2% FCS (Invitrogen) and tissue aggregates were eliminated by adding DNase I (30 µl; Sigma) to the solution. The tissue was then gently shaken and a first supernatant was taken off after the pellet was decanted. The tissue was then gently triturated with a fire polished Pasteur pipette and different supernatants were collected until complete dissociation of the retinal tissue. The cell suspension was then centrifuged at 800 rpm for 5 minutes and cells resuspended in NBA (Invitrogen) supplemented with B27 (1:50, Invitrogen) and glutamine (1:100, Invitrogen) (NBA⁺).

For cell panning, glass coverslips were placed into Petri dishes (60 mm; Corning) and incubated for 2 hours at 37°C with a goat anti-rabbit IgG directed against the PNA lectin (1:100; Sigma ; A4404) diluted in 2 ml of Tris-HCl buffer (50 mM; pH 9.5). After 3 washes with warm PBS, coverslips were incubated in the Tris-HCl buffer containing the PNA lectin (1:40; Sigma). After 2 hours at 37°C, coverslips were again washed with PBS and transferred into 2 ml of D-PBS (Gibco) supplemented with BSA (0.2%; Fraction V; Sigma). The retinal suspension obtained was subsequently added onto the panned coverslips at a density of 4.10⁵ cells/cm² in 24-wells culture plates and incubated for 15 min while gently swirling the plates every 5 minutes. Wells were then washed 5 times with NBA in order to remove non adherent cells. Purified cells were finally incubated with either NBA⁺ or conditioned medium obtained from pure retinal Müller glial cell cultures (RMG CM) and maintained *in vitro* until patch-clamp recordings or immunohistochemistry while refreshing mediums every two days.

### Müller glial cell conditioned medium

Conditioned medium (CM) were obtained from purified pig retinal Müller glial cells (RMG) cultured in NBA⁺. Müller cells were isolated following the procedure described by Guidry (1996) from cell suspensions prepared as described above. Briefly, the total retinal cell suspension was laid on a 10 ml continuous density gradient composed of 0-50% Percoll in normal saline and centrifuged for 5 minutes at 1700 rpm. The band located at the middle of the tube, containing the partly purified Müller glial cells, was taken off and submitted to a mild centrifugation (800 rpm for 5 minutes) in DMEM medium (Invitrogen) supplemented with 10% FCS in order to remove the Percoll. The pellet was resuspended in 1 ml DMEM-10% FCS and again centrifuged at 1700 rpm on a second Percoll gradient. The medium band was collected and the Percoll washed out by mild centrifugation.

The purified Müller glial cells were finally seeded at a density of 0.4X10⁵ cells/cm² in 6-well culture plates previously coated with poly-D-Lysine (1:100; Sigma) and laminine (1:200; Sigma) and cultured in DMEM-10% FCS.

24 hours later, cells were washed twice with DMEM-10% FCS in order to remove the remaining Percoll and allowed to grow. When the culture reached half-confluence, the NBA⁺ medium was substituted to the DMEM-10% FCS medium.

The RMG CM was collected every 2 days and immediately added to the lectin-panned cells.

### Histology

Stainings were achieved as described previously (Gaudin *et al.,* 1997; Picaud *et al.,* 1998a,b). Briefly, isolated pig retinae were fixed in 4% paraformaldehyde (PAF) prepared in 0.1 M PBS (pH 7) for 1h and cryoprotected by immersion in sucrose gradients. Retinal sections (8 µm) obtained on a cryostat were permeabilized with 0.1 % Triton-X100 for 5 min. Cultured cells were similarly immersion fixed with 4% PAF for 15 min and permeabilized with 0.1% Triton-X100 for 5 min. In order to prevent unspecific labeling, cultures and retinal sections were incubated 1h in a blocking buffer containing 10% goat serum and 2% bovine serum albumin in 0.01 M PBS.

Staining was achieved with PNA coupled to Alexa 488 (1:40-1:100, L-21409, Molecular Probes), a mouse monoclonal antibody directed against the PKCα (1:100, Sigma), a rabbit polyclonal antibody directed against the human cone arrestin (hCAR, 1:20.000-1:100.000, epitope = QKAVEAEGDEGS), a rabbit polyclonal to human S-cone opsin (AB5407; 1:10.000-1:20.000) and a rabbit polyclonal to human M/L-cone opsin (AB5405; 1:2000-1:5000), both commercially available from Chemicon.

All antibodies were diluted in the blocking buffer solution. For the immunolabeling, cells or retinal sections were incubated with the primary antibodies for 3h at room temperature, rinsed several times with PBS, and incubated at 37°C for 1h with anti-rabbit or anti-mouse IgGs coupled to Alexa-594 (red emission) or Alexa-488 (green emission) (Molecular Probes). The Alexa 488-coupled PNA and the nucleus marker DAPI (1:200, Sigma) were incubated as secondary antibodies.

### Cell counting and statistical analysis

All experiments were realized in triplicates and cell counting was performed on 3 coverslips for each experiment. For each coverslip, cells were counted under the 40X objective in 30 adjacent areas along the diagonals. Results are expressed as ratio of number of hCAR-labeled cells versus number of DAPI-stained nuclei and/or Nomarski counting. Fluorescent labeling was observed using a Nikon Optiphot 2 Microscope under epifluorescence illumination. All images were acquired with a CCD color Cool-Snap FX camera and analyzed with the Metaview software (Roper Scientific Inc.).
The effect of culture mediums on cone photoreceptors survival was assessed by analysis of variance (ANOVA) followed, when appropriate, by 2 X 2 comparisons based on the Newmam-Keuls test (Winer, 1971). All analyses were conducted on data means of 3 coverslips after counting of hCAR-labeled cells under the 20X objective in 30 areas.

### Patch clamp recordings

Cone photoreceptors were recorded with the patch clamp technique in the whole cell mode either freshly after dissociation or following 2 to 5 days in culture. Recording pipettes were pulled from thin-walled borosilicate glass (TW150F, World Precision Instruments) using a Brown and Flaming-type puller (P-87, Sutter Instruments). Cells were voltage clamped using an RK400 amplifier (Biologic). Data were acquired and analyzed using the Patchit and Tack software packages, respectively (Grant and Werblin, 1994).

For *in vitro* recordings, the standard perfusing solution had the following composition (in mM): 135 NaCl, 5 KCl, 1 CaCl₂, 1 MgCl₂, 10 glucose and 5 HEPES, pH adjusted to 7.74 with NaOH and was delivered by a general gravity-driven perfusion system (~2 ml/min) at room temperature. The pipette solution contained (in mM): 140 KCl, 1 MgCl₂, 0.5 EGTA, 5 ATP, and 4 HEPES (pH adjusted to 7.4 with KOH). All chemicals were obtained from Sigma.

For *in situ* recordings, the perfusing solution was an Ames medium (Sigma) continuously bubbled with carbogen to equilibrate pH.

### Retinal slice preparation

Small square pieces (4 X 4 mm²) of fresh pig retina were dissected out and flat mounted, photoreceptor side up, onto filter paper squares of bigger dimension. The whole preparation were immerged in cold Ames medium and 100~150 µm sections were proceeded with a razor blade maintained in a stereotaxic frame. The slices were then transferred into a perfusion chamber and the filter paper incrusted on grease tubes in order to expose the retinal slices horizontally. Cells were filled with the sulforhodamine-101 fluorescent dye (Sigma) during the recording and observed under epifluorescence illumination (red emission). Cone photoreceptors were then counter-stained with PNA coupled to Alexa 488 (green emission).

### Cloning of Sus scrofa arrestin

Total RNA from pig retina was prepared using Trizol reagent (Invitrogen) according to the protocol provided by the manufacturer. A 922 bp pig cone arrestin fragment was first cloned using oligonucleotides whose sequences were based upon the comparison of diverse mammals arrestin sequences. Then the complete pig arrestin 1416nt coding sequence was obtained using Invitrogen 5' and 3' Race strategy: the cDNA sequence for *Sus scrofa* arrestin-C (ARR3 gene) was submitted to EMBL Nucleotide Sequence Database and delivered under the accession number AJ564496 (SEQ ID NO:5; Figure 7A).

### Single-cell RT-PCR

Isolated cells were randomly picked up from coverslips using a patch clamp recording pipette filled with 8µl of buffer solution (in mM): 140 KCl, 1 MgCl₂, 0.5 EGTA, 5 ATP and 4 HEPES (pH 7.4) and then pulled out into a thin wall PCR tube maintained in ice and containing 40 µl of the reaction mix SuperScriptIII One-Step RT-PCR system (Invitrogen) and couples of oligonucleotides. Tubes were placed at -80°C until all the samples for one experiment have been collected. Tubes were allowed to slightly defreeze on ice and 2 µl of SuperScriptIII RT/Platinum Taq mix were added to each sample. The cDNA synthesis was achieved in a 30 min incubation at 50°C followed by one cycle of denaturation at 94°C for 2 min, 37 cycles of a PCR amplification (94°C for 30", 52°C for 45", 68°C for 45") and a final extension of one cycle at 68°C for 5 min. A dilution of the original PCR was re-amplified using different couples of oligonucleotides (see below) : 5 µl aliquot of the primary PCR was diluted into 495 µl TE buffer. Five µl of this diluted primary PCR product were used for a second Taq PCR (Invitrogen) cycle made of one cycle of denaturation at 94°C for 2 min followed by 35 cycles (94°C 30", 55°C 45", 72°C 45") and one cycle at 72°C for 5 min. Ten µl of the PCR product were analyzed on ethidium bromide staining agarose gel.

The sequences of oligonucleotides used for the first single-cell RT-PCR were as follow:

+205 [GGGAAACGGACTTCGTG] +222 SEQ ID NO:1

+593 [GCACAGAAACTCTTCACTTC] +574 SEQ ID NO:2

The 389 bp fragment obtained from the primary RT-PCR was then amplified using the following oligonucleotides:

+ 219 [CGTGGACCATGTGGACATG] +237 SEQ ID NO:3

+509 [AGGTTGACAACCATCTGCAG] +490 SEQ ID NO:4

### Results

### Lectin-panning of photoreceptors : immunohistochemistry

We assessed whether the lectin PNA could promote the selective adherence of this cell population to a culture plate, whereas the cells had been subjected to enzymatic and mechanic treatments.

When cells were observed immediately after the lectin-panning procedure, most had the typical morphology of cone photoreceptors with a short outer segment, inner segment and cell body of similar sizes and a long axon ending by a large pedicle (Fig. 1A-B). When such cells were immunolabeled with the human anti-arrestin antibody (hCAR) that labels the entire cone cell cytoplasm in the pig retina (Fig. 2E), most cells appeared immunopositive (Fig. 1C). This observation indicated that cone photoreceptors can be purified by PNA-lectin-panning.

Since PNA was reported to bind differently to the different populations of cone photoreceptors (Ebrey and Koutalos, 2001), we verified that the lectin-panning procedure allowed the purification of all cone types. In the pig retina, PNA stained intensely the outer and inner segments of cone photoreceptors, more lightly their cell bodies located in the outer row of the outer nuclear layer (ONL), and heavily their axons and pedicles (Fig. 2B). Immunolabeling with the human cone arrestin antibody (hCAR) showed a complete colocalization with the PNA-lectin staining suggesting that both cone photoreceptor cell populations were stained by PNA in the pig retina. When sections were immunolabeled with the blue (Short wavelength, S-) or the red/green (Middle to Long wavelength, M/L-) cone opsin antibodies (Fig. 2G-L), both types of cone photoreceptors were stained by PNA. In the lectin-panning culture, both S- and L/M-opsin immunopositive cells were presents (Fig. 1E-F). These observations indicated that PNA lectin-panning can select both S- and L/M-cone photoreceptors.
After two days in culture, lectin-panned cells lost their outer and inner segments and acquired an oval-shaped and medium-sized cell bodies with short neuritis (Fig. 3B). When these cultures were labeled with the cone arrestin antibody 24-48h after the dissociation, all cells remained arrestin-immunopositives (Fig. 3B). Cone arrestin-positive cells accounted for 92.57 ± 2.12 % (s.e.m., *n* = 9) of cells observed under transmitted light (Fig. 3C). To further assess the cone quantification, cultured cells were labeled with the nuclear dye DAPI (Fig. 1D). In this case, cone arrestin positive photoreceptors accounted for 80.2 ± 2.32 % (s.e.m., *n* = 9) of DAPI-stained nuclei (Fig. 3A). The difference between the two counts may arise from DAPI-labeling of degenerated cells not taken into account in the first quantification. To identify contaminating cells, cultured cells were immunolabeled with the PKCα antibody which selectively labels rod bipolar cells (Karschin and Wässle, 1990). Our observations indicated that rod bipolar cells accounted for 5.58 ± 0.05 % (s.e.m., *n* = 3) of DAPI-stained nuclei. Very few glial cells were detected (identification by their large DAPI-stained cell bodies). These observations indicated that lectin-panning give rise to a high degree of purification of cone photoreceptors in culture.

### Lectin-panning of photoreceptors : single-cell RT-PCR

Adherent cells were picked up from 3 independent set of experiments. In the first experiment, 15 cells were collected immediately after the panning procedure. In a second experiment, 17 cells were picked up three days after the lectin-panning and in the last experiment, 15 cells were withdrawn five days after their adhesion.

For each experiment, sources of contamination such as RT-PCR buffers, intra-cellular pipette buffer and perfusing solution (Fig. 4) were checked. In order to detect a possible genomic contamination, the Super-Script reverse transcriptase was omitted in three tubes for each experiment and replaced by Taq Platinum alone. The oligonucleotides used in this study were carefully designed and contained no less than seven mismatches with sequences located in the homologous regions of *Sus scrofa* S-antigen mRNA (accession number S82664 ; SEQ ID NO:7 ; *cf.* Figure 7B). If any noticeable signal was absent in the different negative controls performed so far, an expected 291bp product corresponding to the cone arrestin fragment was observed in most of the single-cell RT-PCR samples analysis. Positive signals observed in the first, second and third experiments were 12 out of 12, 13 out of 14 and 10 out of 12 respectively, corresponding to a 92.06 ± 4.82 % purity of the preparation (s.e.m., *n* = 3).

### Glial dependence of cone photoreceptor survival

It was previously reported that Müller glial cells are required for the survival of adult pig photoreceptors isolated by vibratome sectioning of the retina (Picaud *et al.,* 1998b). To assess whether cone photoreceptor rely on glial neurotrophic factors for their survival, cone photoreceptor cells isolated by PNA lectin-panning were cultured in the presence of glia conditioned medium (RMG CM, see material and methods). After 24h in culture, no clear difference was observed in the number of cone photoreceptors between the RMG CM and control conditions. By contrast, after 4 days in culture a major difference was observed between the two mediums. While the number of hCAR-positive cells decreased by 69.41 ± 8.97 % in control conditions (s.e.m., *n* = 12), it remained stable in the RMG CM, a 2.59 decrease (± 9.44 %; s.e.m., *n* = 12) was only observed. After one week in vitro culture, the survival effect produced by the RMG CM was even more pronounced. The number of cone photoreceptors decreased by 96.48 ± 0.65 % (s.e.m., *n* = 12) in control conditions whereas it only decreased by 31.10 ± 8.13 % in RMG CM (s.e.m., *n* = 12) (Figure 5E). These differences between the glia conditioned medium and control conditions at 4 days and at 1 week in culture were statistically significant at p < 0.05. These results indicated that cultured glial cells can release molecules facilitating cone photoreceptor survival in culture.

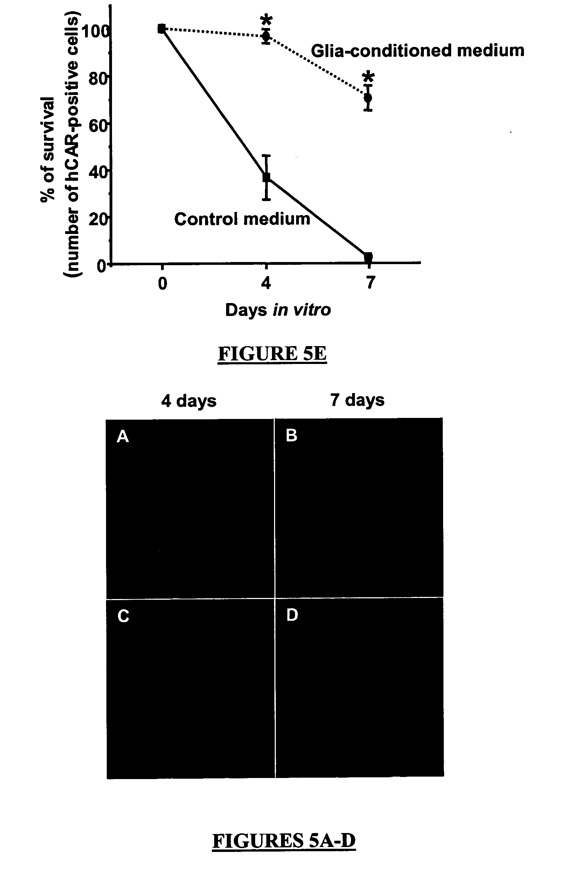

When looking at morphological appearance of cells isolated by the PNA lectin-panning method no drastic differences appeared at 24h between cells cultured in either control conditions or RMG CM. In both cases, cells had a rounded appearance with medium sized cell bodies showing sometimes short neuritis. However, after 4 days in culture, the number of cells developing neuritis was increased in RMG CM medium when compared to NBA⁺ medium (Figures 5A-D). These processes were still observed in cone photoreceptors cultured for 1 week in glia-conditioned media (Figure 5 D). These results suggested that retinal Müller glial cells can release diffusible factors promoting both adult cone photoreceptor survival and development *in vitro.*

### Intrinsic electrophysiological homogeneity of cultured cells

To characterize the cone photoreceptor physiology, we used the whole cell configuration of the patch clamp technique to study the voltage-dependant activated currents in theses cells either *in situ* or after the panning procedure. Figure 6A illustrates a sample trace of a typical cone recorded in a pig retinal slice. Cone photoreceptors showed a slowly-activating inward current at hyperpolarizing potentials (Vₕ = -70 mV, Iₘₐₓ = - 58 ± 9 pA; Vₕ = -120 mV, Iₘₐₓ = -329 ± 19 pA, n = 32) rising a peak amplitude within 50 msec (Fig. 6B). With a standard intracellular solution, the total current reversed near -40 mV. Outward currents activated at depolarizing potentials were of small amplitude (Vₕ = +50 mV, Iₘₐₓ = -575 ± 47 pA, *n* = 32), linear above -20 mV, and did not decayed with time. To assess their cell physiology *in vitro,* cone photoreceptors isolated by lectin-panning were recorded after the isolation. These cells expressed voltage-gated conductances similar to that of cone photoreceptors *in situ* excepted a slight increase in the whole cell conductances recorded at very hyperpolarized potentials. Recordings were obtained from 3 different cultures. These results confirmed that lectin-panned cells were viable and had physiological features of cone photoreceptors.

### Discussion

We selected and assayed peanut agglutinin lectin (PNA) to design a procedure to purify selectively an adult cone cell population. The purification used PNA-coated cell culture plates. Incubation of a retinal cell suspension from the pig retina on this PNA-coated plates promoted the selective adherence of both S and L/M cone photoreceptors at a purity grade superior to 80%.

When Müller glial cell conditioned medium was incubated on lectin-purified cells, cone photoreceptor survival increased from 4 to 69% at 1 week. This study not only provides a new model to screen neuroprotective molecules for cone photoreceptors but it also open new possibilities for the characterization of cone photoreceptors in normal and pathological conditions.

### Cone photoreceptor cell sorting

The first attempts relating to cone isolation used chick embryo retina, which were in vitro cultured on low-density glia-free monolayers, and used to investigate retinal cell differentiation (Adler *et al.,* 1984; see also review by Adler, 2000). For instance, retinal cells already postmitotic at embryonic day 5 (ED5) were shown to be almost 70% to differentiate as photoreceptors cells when cultured at ED6 whereas they were only 35% when isolated at ED8 (Adler and Hatlee, 1989).

Vibratome sectioning (Silverman and Hughes, 1989) and laser dissection (Salchow *et al.,* 2001) of the retina has also been used in an attempt to purify photoreceptors. In culture, such isolated photoreceptors were found to survive for only a few days when prepared from young rat retina (Fontaine *et al.,* 1998) or from embryonic human retina (Salchow *et al.,* 2001). However, at the adult stage, such isolated photoreceptors required a glial feeder layer to survive (Picaud *et al.,* 1998).

Finally, a progressive mechanic dissociation of enzymatically-treated retina also provided cultures enriched in photoreceptors (95%), but with a cone/photoreceptor ratio between 35-45% depending on the experimental conditions (Traverso *et al.,* 2003).

The technique described in our study generated a 92% purity of adult fully differentiated cone photoreceptors. Cone cell identity was verified by their electrophysiological signature and by molecular markers, such as the cone arrestin or cone opsins, which were revealed at either gene expression with single RT-PCR or the protein with immunohistochemistry. The purity of the preparation appeared less important when cone cells were referred to the total number of DAPI-labeled cell nuclei rather than to the number of cells observed under transmitted light. This difference may rely on an overestimation of DAPI-labeled cell nuclei that are likely to include nuclear remnants of degenerated cells not identified as cells under transmitted light. PNA lectin-panning can therefore provide a high degree of purification of adult cone photoreceptors from retina.

The peanut agglutinin lectin (PNA) is typically considered a valuable marker of the extracellular matrix domain surrounding cone photoreceptor outer and inner segments (Ahnelt and Kolb, 2000). However, depending on species, the lectin labeling was not always identical depending on the cone spectral sensitivity thus revealing different compositions of their extracellular matrix domains. In ground squirrel, for instance, S-cones labeling is more intense than that of L-cones (Röhlich *et al.,* 1989; Szél *et al.,* 1993) while in fish and frog retina, PNA identifies L-cones selectively (Ishikawa *et al.,* 1997). In the primate retina *(Microcebus murinus),* both S- and M/L-cones are decorated around their outer and inner segments, with an additional weak staining around their cell body and at their cone pedicle (Dkhissi-Benyahya *et al.,* 2001). These observations are generally confirmed in most mammalian species like mouse and rats with intense labeling around cone outer/inner segments and at cone pedicles (Blanks and Johnson, 1983; Fei, 2003). In the pig retina, the labeling was similarly observed around both S- and M/L-cones (Fig. 2).

Cone photoreceptor labeling is not only observed in normal conditions but also in pathological states. Following rod photoreceptor loss and during their own secondary degeneration, cone cells were indeed identified and quantified by PNA labeling in the rd1 mouse retina of the living animal (Mohand Said *et al.,* 1997) or following explant culture (Mohand-Said *et al.,* 1998).

The present results demonstrate that PNA can nevertheless be successfully used to mediate isolation of cone photoreceptor cells from an adult mammalian retinal tissue, and that PNA binding can still be operative and selective even though the tissue to which it is applied has undergone extracellular matrix dissociation.

FACS technique using fluorescent PNA lectin is much likely to be very efficient in sorting cone photoreceptors.

### Glial neuroprotection of cone photoreceptors

In culture, the absence of glial cells did not prevent chick embryo cone photoreceptors to survive (Adler *et al.,* 1984). By contrast, glial cells appeared very important for the survival of neonatal and adult mammalian rod photoreceptors (Kjavin and Reh, 1991; Hicks *et al.,* 1994). The absolute glial dependence of photoreceptors was demonstrated when rods and cones isolated by vibratome sectionning the outer retina were found to survive only on a glial feeder layer (Picaud *et al.,* 1998). This glial cell dependence of photoreceptors was further supported by the neuroprotective effect of neurotrophic factors like BDNF, GDNF, CNTF, that do not seem to have functional receptors on photoreceptors but instead on glial cells (Ugolini *et al.,* 1995; Kirsch *et al.,* 1998; Harada *et al.,* 2003). Photoreceptor survival would indeed rely on Müller cell activation (Wahlin *et al.,* 2000; Harada *et al.,* 2003). Our results clearly establish that cone photoreceptors are dependent upon a diffusible trophic molecule released by retinal glial cells for their survival.

Retinal glial cells were reported to contain, and synthesize many growth factors and cytokines (Hicks *et al.,* 1991; Mascarelli *et al.,* 1991; La Vail *et al.,* 1992; Li *et al.,* 1995, 1997; Milam *et al.,* 1997). The notion that glial cells synthesize trophic factors for retinal cells is consistent with the survival and outgrowth of retinal ganglion cells in the presence of a glia-conditioned medium (García *et al.,* 2002). It is also in agreement with the BDNF, GDNF and CNTF neuroprotection of photoreceptors that may require Müller cell activation (see above). Future studies should therefore focuss on the isolation of such glia-derived trophic molecules.

### Conclusions

The present study provides a strategy to isolate cone photoreceptors that is very valuable to characterize cone photoreceptor at a genomic and proteomic level in both normal and pathological conditions. Already, its use in culture demonstrated the glial dependence of cone photoreceptors opening the search for glia-derived cone trophic factors.

### BIBLIOGRAPHIC REFERENCES:

Adler R. A model of retinal cell differentiation in the chick embryo. *Prog Retin Eye Res.* 2000;19:529-57.
Adler R, Hatlee M. Plasticity and differentiation of embryonic retinal cells after terminal mitosis. *Science.* 1989;243:391-3.
Adler R, Lindsey JD, Elsner CL. Expression of cone-like properties by chick embryo neural retina cells in glial-free monolayer cultures. *J Cell Biol.* 1984;99:1173-8.
Ahnelt PK, Kolb H. The mammalian photoreceptor mosaic-adaptive design. *Prog Retin Eye Res.* 2000;19:711-77.
Blanks JC, Johnson LV. Selective lectin binding of the developing mouse retina. *J Comp Neurol.* 1983;221:31-41.
Chandler MJ, Smith PJ, Samuelson DA, MacKay EO. Photoreceptor density of the domestic pig retina. *Vet Ophthalmol.* 1999;2:179-184.
Dkhissi-Benyahya O, Szel A, Degrip WJ, Cooper HM. Short and mid-wavelength cone distribution in a nocturnal Strepsirrhine primate (Microcebus murinus). *J Comp Neurol.* 2001;438:490-504.
Ebrey T, Koutalos Y. Vertebrate photoreceptors. *Prog Retin Eye Res.* 2001;20:49-94.
Fei Y. Development of the cone photoreceptor mosaic in the mouse retina revealed by fluorescent cones in transgenic mice. *Mol Vis.* 2003;9:31-42.
Fintz AC, Audo I, Hicks D, Mohand-Said S, Leveillard T, Sahel J. Partial characterization of retina-derived cone neuroprotection in two culture models of photoreceptor degeneration. *Invest Ophthalmol Vis Sci.* 2003;44:818-25.
Fontaine V, Kinkl N, Sahel J, Dreyfus H, Hicks D. Survival of purified rat photoreceptors in vitro is stimulated directly by fibroblast growth factor-2. *J Neurosci.* 1998;18:9662-72.
Garcia M, Forster V, Hicks D, Vecino E. Effects of muller glia on cell survival and neuritogenesis in adult porcine retina in vitro. *Invest Ophthalmol Vis Sci.* 2002;43:3735-43.
Gaudin C, Forster V, Sahel J, Dreyfus H, Hicks D. Survival and regeneration of adult human and other mammalian photoreceptors in culture. *Invest Ophthalmol Vis Sci.* 1996;37:2258-68.
Geller AM, Sieving PA. Assessment of foveal cone photoreceptors in Stargardt's macular dystrophy using a small dot detection task.
   Vision Res. 1993 Jul;33(11):1509-24.
Guidry C. Isolation and characterization of porcine Muller cells. Myofibroblastic dedifferentiation in culture. *Invest Ophthalmol Vis Sci.* 1996;37:740-52.
Harada C, Harada T, Quah HM, Maekawa F, Yoshida K, Ohno S, Wada K, Parada LF, Tanaka K. Potential role of glial cell line-derived neurotrophic factor receptors in Muller glial cells during light-induced retinal degeneration. *Neuroscience.* 2003;122:229-35.
Hendrickson A, Hicks D. Distribution and density of medium- and short-wavelength selective cones in the domestic pig retina. *Exp Eye Res.* 2002;74:435.
Hicks D, Forster V, Dreyfus H, Sahel J. Survival and regeneration of adult human photoreceptors in vitro. *Brain Res.* 1994;643:302-5.
Ishikawa M, Hashimoto Y, Tonosaki A, Sakuragi S. Preference of peanut agglutinin labeling for long-wavelength-sensitive cone photoreceptors in the dace retina. *Vision Res.* 1997;37:383-7.
Karschin A, Wassle H. Voltage- and transmitter-gated currents in isolated rod bipolar cells of rat retina. *J Neurophysiol.* 1990;63:860-76.
Kirsch M, Schulz-Key S, Wiese A, Fuhrmann S, Hofmann H. Ciliary neurotrophic factor blocks rod photoreceptor differentiation from postmitotic precursor cells in vitro. *Cell Tissue Res.* 1998;291:207-16.
Kivela T, Tarkkanen A. A lectin cytochemical study of glycoconjugates in the human retina. *Cell Tissue Res.* 1987;249:277-88.
Kljavin IJ, Reh TA. Muller cells are a preferred substrate for in vitro neurite extension by rod photoreceptor cells. *J Neurosci.* 1991;11:2985-94.
La Vail MM, Unoki K, Yasumura D, Matthes MT, Yancopoulos GD, Steinberg RH. Multiple growth factors, cytokines, and neurotrophins rescue photoreceptors from the damaging effects of constant light. *Proc Natl Acad Sci U S A.* 1992;89:11249-53.
Li ZY, Chang JH, Milam AH. Distribution of basic fibroblast growth factor in human retinas with retinitis pigmentosa. *Exp Eye Res.* 1997;65:855-9.
Li ZY, Chang JH, Milam AH. A gradient of basic fibroblast growth factor in rod photoreceptors in the normal human retina. *Vis Neurosci.* 1997;14:671-9.
Li ZY, Kljavin IJ, Milam AH. Rod photoreceptor neurite sprouting in retinitis pigmentosa. *J Neurosci.* 1995;15:5429-38.
Mascarelli F, Tassin J, Courtois Y. Effect of FGFs on adult bovine Muller cells: proliferation, binding and internalization. *Growth Factors.* 1991;4:81-95.
Milam AH, Li ZY, Fariss RN. Histopathology of the human retina in retinitis pigmentosa. *Prog Retin Eye Res.* 1998;17:175-205.
Mohand-Said S, Deudon-Combe A, Hicks D, Simonutti M, Forster V, Fintz AC, Leveillard T, Dreyfus H, Sahel JA. Normal retina releases a diffusible factor stimulating cone survival in the retinal degeneration mouse. *Proc Natl Acad Sci U SA.* 1998;95:8357-62.
Mohand-Said S, Hicks D, Simonutti M, Tran-Minh D, Deudon-Combe A, Dreyfus H, Silverman MS, Ogilvie JM, Tenkova T, Sahel J. Photoreceptor transplants increase host cone survival in the retinal degeneration (rd) mouse. *Ophthalmic Res.* 1997;29:290-7.
Picaud S, Hicks D, Forster V, Sahel J, Dreyfus H. Adult human retinal neurons in culture: Physiology of horizontal cells. *Invest Ophthalmol Vis Sci.* 1998;39:2637-48.
Picaud S, Pattnaik B, Hicks D, Forster V, Fontaine V, Sahel J, Dreyfus H. GABAA and GABAC receptors in adult porcine cones: evidence from a photoreceptor-glia co-culture model. *JPhysiol.* 1998;513 (Pt 1):33-42.
Rohlich P, Szel A, Johnson LV, Hageman GS. Carbohydrate components recognized by the cone-specific monoclonal antibody CSA-1 and by peanut agglutinin are associated with red and green-sensitive cone photoreceptors. *J Comp Neurol.* 1989;289:395-400.
Rohlich P, Szel A, Papermaster DS. Immunocytochemical reactivity of Xenopus laevis retinal rods and cones with several monoclonal antibodies to visual pigments. *J Comp Neurol.* 1989;290:105-17.
Salchow DJ, Trokel SL, Kjeldbye H, Dudley T, Gouras P. Isolation of human fetal cones. *Curr Eye Res.* 2001;22:85-9.
Silverman MS, Hughes SE. Transplantation of photoreceptors to light-damaged retina. *Invest Ophthalmol Vis Sci.* 1989;30:1684-90.
Szel A, Rohlich P, Mieziewska K, Aguirre G, van Veen T. Spatial and temporal differences between the expression of short- and middle-wave sensitive cone pigments in the mouse retina: a developmental study. *J Comp Neurol.* 1993;331:564-77.
Szel A, Rohlich P, Van Veen T. Short-wave sensitive cones in the rodent retinas. *Exp Eye Res.* 1993;57:503-5.
Szel A, von Schantz M, Rohlich P, Farber DB, van Veen T. Difference in PNA label intensity between short- and middle-wavelength sensitive cones in the ground squirrel retina. *Invest Ophthalmol Vis Sci.* 1993;34:3641-5.
Traverso V, Kink1 N, Grimm L, Sahel J, Hicks D. Basic fibroblast and epidermal growth factors stimulate survival in adult porcine photoreceptor cell cultures. *Invest Ophthalmol Vis Sci.* 2003;44:4550-8.
Travis GH. Mechanisms of cell death in the inherited retinal degenerations. *Am J Hum Genet.* 1998;62:503-8.
Uehara F, Yanagita T, Iwakiri N, Ohba N. Immunohistochemical localization of MUC1 glycoprotein in the retina. Jpn J Ophthalmol. 1997 May-Jun;41(3):200-1.
Ugolini G, Cremisi F, Maffei L. TrkA, TrkB and p75 mRNA expression is developmentally regulated in the rat retina. *Brain Res.* 1995;704:121-4.
Wahlin KJ, Campochiaro PA, Zack DJ, Adler R. Neurotrophic factors cause activation of intracellular signaling pathways in Muller cells and other cells of the inner retina, but not photoreceptors. *Invest Ophthalmol Vis Sci.* 2000;41:927-36.
Yan Q, Bumsted K, Hendrickson A. Differential peanut agglutinin lectin labeling for S and L/M cone matrix sheaths in adult primate retina. *Exp Eye Res.* 1995;61:763-6.

## Claims

1. Process for the *in vitro* production of a population of cone photoreceptors by isolation from a retinal tissue with a cone purity of at least 80%, typically of about 90%, said process promoting no selection of the S-cone sub-population *vs*. the L/M-cone sub-population, wherein said process comprises:
- optionally, chopping the retinal tissue into small fragments,
- dissociating the extracellular matrix of the retinal tissue or retinal tissue fragments enzymatically and/or mechanically and/or chemically, so as to dissociate the retinal cells from each other without lysing the retinal cells,
- optionally, suspending the dissociated cells in a solution,
- placing said dissociated cells in contact with PNA (peanut germ agglutinin) under conditions enabling cell-PNA binding,
- recovering the PNA-bound cells, and optionally *in vitro* culturing the PNA-bound cells,
whereby said population of photoreceptor cones is obtained.

2. Process according to claim 1, **characterized in that** said retinal tissue is an adult tissue.

3. Process according to any one of the preceding claims, **characterized in that** said retinal tissue is a mammalian retinal tissue.

4. Process according to any one of claims 1-3, **characterized in that** said retinal tissue is a human retinal tissue.

5. Process according to any one of claims 1-3, **characterized in that** said retinal tissue is a animal but non-human retinal tissue, such as a pig, rat or mouse retinal tissue.

6. Process according to any one of the preceding claims, **characterized in that** said retinal tissue is a pathologic retinal tissue that undergoes or has undergone photoreceptor degeneration.

7. Process according to claim 6, **characterized in that** said pathologic retinal tissue originates from an individual afflicted with an inherited or acquired disease involving photoreceptor degeneration, said disease being selected from the group consisting of retinitis pigmentosa and age macular degeneration.

8. Process according to any one of the preceding claims, **characterized in that** said dissociation of the extracellular matrix of the retinal tissue or retinal tissue fragments comprises an enzymatic dissociation performed with a proteolytic enzyme.

9. Process according to claim 8, **characterized in that** said proteolytic enzyme is papain.

10. Process according to claim 8, **characterized in that** said proteolytic enzyme is trypsin.

11. Process according to any one of claims 8-10, **characterized in that** said dissociation further comprises a mechanical dissociation.

12. Process according to any one of the preceding claims, **characterized in that** said PNA is bound to a solid support.

13. Process according to any one of the preceding claims, **characterized in that** said placing in contact of the dissociated retinal cells with PNA is achieved by panning, MACS (magnetic activated cell sorting), or FACS (fluorescent activated cell sorting technique).

14. Process according to any one of the preceding claims, **characterized in that** it comprises *in vitro* culturing the PNA-bound cells on a conditioned medium obtainable by collection of the culture medium after *in vitro* culture of Müller glial cells.

15. Process according to claim 14, **characterized in that** said conditioned medium is obtainable by collection of the culture medium after *in vitro* culture of Müller glial cells on NBA™ (Neurobasal Medium), optionally supplemented by B27 and glutamine.

16. A population of cone photoreceptors obtainable by the process according to any one of claims 1 to 15, **characterized in that** it has a purity of at least 80% in cone cells.

17. A population of cone photoreceptors according to claim 16, **characterized in that** said cone cells are adult cone cells.

18. A population of cone photoreceptors according to any one of claims 16-17, **characterized in that** said cone cells are mammalian cone cells.

19. A population of cone photoreceptors according to any one of claims 16-18, **characterized in that** said cone cells are human cone cells.

20. A population of cone photoreceptors according to any one of claims 16-18, **characterized in that** said cone cells are animal but non-human cone cells, such as pig, rat or mouse cone cells.

21. A population of cone photoreceptors according to any one of claims 16-20, **characterized in that** it comprises both S-cone and L/M-cone photoreceptors.

22. Use of a conditioned culture medium obtainable by collection of the culture medium after *in vitro* culture of Müller glial cells, for the *in vitro* culture of cone cells.

23. Process for the production of a culture medium that is adapted to the *in vitro* culture of a population of cone photoreceptors, such as the population according to any one of claims 16-21, said process comprising conditioning a culture medium that is suitable for cone survival with Müller glial cells, by culturing Müller glial cells onto a culture medium that is suitable for cone survival, and collecting the resulting culture medium, this collected culture medium being is adapted to the *in vitro* culture of a population of cone photoreceptors, such as the one according to any one of claims 16-21.

24. Process for the production of a culture medium according to claim 23, **characterized in that** the pre-conditioned culture medium that is selected as being favourable to cone survival and that is intended to receive said Müller glial cells for culture is NBA™ culture medium, optionally supplemented by B27 and glutamine, or DMEM/F12 or Ames.

25. Cell culture which comprises:
- a population of cone photoreceptors according to any one of claims 16-21, and
- a culture medium obtainable by the process of claim 23 or 24.

26. Multi-well culture plate, comprising in at least one of its wells, a population of cone photoreceptors according to any one of claims 16-21, and optionally a culture medium obtainable by the process of claim 23 or 24.

27. Kit for the *in vitro* production of a population of cone photoreceptors by isolation from a retina tissue with a cone purity of at least 80%, typically of about 90%, said kit comprising:
- a proteolytic enzyme, and
- PNA, optionally bound on a solid support,
- and optionally, a culture medium intended for the *in vitro* culture of said cone cells, this culture medium being obtainable by collection of the culture medium after *in vitro* culture of Müller glial cells.

28. Kit according to claim 27, **characterized in that** said proteolytic enzyme is papain or trypsin.

29. Method of screening for compounds capable of showing protective and/or anti-degenerative properties on cone photoreceptors, **characterized in that** it comprises:
- providing a population of cone photoreceptors that undergoes or has undergone cone degeneration, using the process according to any one of claims 6-7, optionally placing this population in the wells of a multi-well culture plate,
- placing said population of cone photoreceptors in contact with a candidate compound,
- assessing whether said candidate compound induces a protective and/or anti-degenerative effect on said population of cone photoreceptors, e.g. by assessing whether it promotes cone cell viability or reduces cone cell mortality.

30. Method according to claim 29, **characterized in that** said population of cone photoreceptors is a population of animal but non-human animal cone photoreceptors, such as pig, rat or mouse cone photoreceptors.

31. Method according to claim 29, **characterized in that** said population of cone photoreceptors is a population of human cone photoreceptors.


SEQUENCE LISTING

<110>  INSERM

<120>  Isolation and culture of a high purity population of cone
       photoreceptor cells, and biological applications thereof

<130>  FP - BEP INSERM Cone Photoreceptors

<160>  8

<170>  PatentIn version 3.2

<210>  1
<211>  18
<212>  DNA
<213>  Artificial Sequence

<220>
<223>  Primer

<400>  1
gggaaacggg acttcgtg                                                    18

<210>  2
<211>  20
<212>  DNA
<213>  Artificial Sequence

<220>
<223>  primer

<400>  2
gcacagaaac tcttcacttc                                                  20

<210>  3
<211>  19
<212>  DNA
<213>  Artificial Sequence

<220>
<223>  primer

<400>  3
cgtggaccat gtggacatg                                                   19

<210>  4
<211>  20
<212>  DNA
<213>  Artificial Sequence

<220>
<223>  primer

<400>  4
aggttgacaa ccatctgcag                                                  20

<210>  5
<211>  1416
<212>  DNA
<213>  Sus scrofa

<220>

<221>  CDS
<222>  (142)..(1326)

<400>  5
agcctgttct tctcccctgg acccccacca atctaaaaac tcagaggacc ttgggtataa      60

gaggttgggc gggcgagcat agcaaccaga gctggagacg gatgtgagct tcatcttact     120

ccccagctca acatcaacac a atg gcc aac atg tcc agg gtg ttt aag aag       171
                        Met Ala Asn Met Ser Arg Val Phe Lys Lys
                        1               5                   10

acc tgc tcc aac ggc aag ctc tcc atc tac ctg ggg aaa cgg gac ttc       219
Thr Cys Ser Asn Gly Lys Leu Ser Ile Tyr Leu Gly Lys Arg Asp Phe
                15                  20                  25

gtg gac cat gtg gac atg gtg gaa ccc att gat ggt gta gtc ctg gtt       267
Val Asp His Val Asp Met Val Glu Pro Ile Asp Gly Val Val Leu Val
            30                  35                  40

gac cct gag tat tta aaa ggt cga aag atg ttt gtc atg ttg aca tgt       315
Asp Pro Glu Tyr Leu Lys Gly Arg Lys Met Phe Val Met Leu Thr Cys
        45                  50                  55

gcc ttt cgc tat ggc cat gat gac ttg gat gtg att ggc ctg aca ttc       363
Ala Phe Arg Tyr Gly His Asp Asp Leu Asp Val Ile Gly Leu Thr Phe
    60                  65                  70

cgc aaa gat ctg tat gta cag gtc cag caa gtg gtc cca gct gag ccc       411
Arg Lys Asp Leu Tyr Val Gln Val Gln Gln Val Val Pro Ala Glu Pro
75                  80                  85                  90

acc agc cct cag gtg ccc ctc acg gtc ttg cag gag cga ctg ctg cac       459
Thr Ser Pro Gln Val Pro Leu Thr Val Leu Gln Glu Arg Leu Leu His
                95                  100                 105

aag ctg ggg gac aat gct tac ccc ttt aac ctg cag atg gtt gtc aac       507
Lys Leu Gly Asp Asn Ala Tyr Pro Phe Asn Leu Gln Met Val Val Asn
            110                 115                 120

ctg ccc tgt tca gtg aca ctg cag cca ggt cct gat gat aca gga aag       555
Leu Pro Cys Ser Val Thr Leu Gln Pro Gly Pro Asp Asp Thr Gly Lys
        125                 130                 135

gcc tgt ggc att gac ttt gaa gtg aag agt ttc tgt gct gaa aac ctg       603
Ala Cys Gly Ile Asp Phe Glu Val Lys Ser Phe Cys Ala Glu Asn Leu
    140                 145                 150

gag gag aaa gtc tcc aag aaa gat tcc gtg cgg ctg gtg att cgg aaa       651
Glu Glu Lys Val Ser Lys Lys Asp Ser Val Arg Leu Val Ile Arg Lys
155                 160                 165                 170

ata cag ttt gca cct gtg gag cca ggc cct ggc ccc tgg gcc cag acc       699
Ile Gln Phe Ala Pro Val Glu Pro Gly Pro Gly Pro Trp Ala Gln Thr
                175                 180                 185

gtt cgc cgt ttc ctt ctg tca gct cag ccc cta caa ctc cag gcc tgg       747
Val Arg Arg Phe Leu Leu Ser Ala Gln Pro Leu Gln Leu Gln Ala Trp
            190                 195                 200

atg gac agg gag gtt cac tac cat ggc aaa cca atc tct gtc aat gtt       795
Met Asp Arg Glu Val His Tyr His Gly Lys Pro Ile Ser Val Asn Val
        205                 210                 215

tcc atc aac aac tcc acc aac aag gtc atc aaa aaa atc aag atc tct       843
Ser Ile Asn Asn Ser Thr Asn Lys Val Ile Lys Lys Ile Lys Ile Ser
    220                 225                 230

gtt gac cag atc aca gat gtt gtc ctg tat tca cta gac aag tac acc     891
Val Asp Gln Ile Thr Asp Val Val Leu Tyr Ser Leu Asp Lys Tyr Thr
235                 240                 245                 250

aag act gtg ttc att cag gag ttc acg gag act ata gct gct aac tcc     939
Lys Thr Val Phe Ile Gln Glu Phe Thr Glu Thr Ile Ala Ala Asn Ser
                255                 260                 265

acc ttc tcc aag agc ttt gaa gta acc cca ctc ctg gct gac aac tgc     987
Thr Phe Ser Lys Ser Phe Glu Val Thr Pro Leu Leu Ala Asp Asn Cys
            270                 275                 280

gag aaa cag ggc ctg gcg ctg gat ggc aaa ctc aag cat ggt gac acc     1035
Glu Lys Gln Gly Leu Ala Leu Asp Gly Lys Leu Lys His Gly Asp Thr
        285                 290                 295

aac ctg gcc tct agc aca att ctt cgg cca gga atg gat aag gag ctg     1083
Asn Leu Ala Ser Ser Thr Ile Leu Arg Pro Gly Met Asp Lys Glu Leu
    300                 305                 310

ctg ggg atc ttg gtg tcc tac aaa gtc aga gtc aac ctg atg gtg tcc     1131
Leu Gly Ile Leu Val Ser Tyr Lys Val Arg Val Asn Leu Met Val Ser
315                 320                 325                 330

tgt ggc ggc atc cta ggc gac ctg aca gcc agt gat gtc ggc gtg gag     1179
Cys Gly Gly Ile Leu Gly Asp Leu Thr Ala Ser Asp Val Gly Val Glu
                335                 340                 345

ctg ccc ctg atc cta atg cac cca aag cca tca aat gag gct gct agc     1227
Leu Pro Leu Ile Leu Met His Pro Lys Pro Ser Asn Glu Ala Ala Ser
            350                 355                 360

tct gag gac ata gtc atc gag gag ttt gct cgg cag gag ccc ggt ggc     1275
Ser Glu Asp Ile Val Ile Glu Glu Phe Ala Arg Gln Glu Pro Gly Gly
        365                 370                 375

aga gaa gag agc cag gag gct ttg gcg gct gag ggg gat gag ggc agc     1323
Arg Glu Glu Ser Gln Glu Ala Leu Ala Ala Glu Gly Asp Glu Gly Ser
    380                 385                 390

tga gcacctagct ctgtgcctct gtgtgggagc cccactgtaa cattctaata     1376

aatcagcttg tccagaaaaa aaaaaaaaaa aaaaaaaaaa     1416

<210>  6
<211>  394
<212>  PRT
<213>  Sus scrofa

<400>  6

Met Ala Asn Met Ser Arg Val Phe Lys Lys Thr Cys Ser Asn Gly Lys
1               5                   10                  15

Leu Ser Ile Tyr Leu Gly Lys Arg Asp Phe Val Asp His Val Asp Met
            20                  25                  30

Val Glu Pro Ile Asp Gly Val Val Leu Val Asp Pro Glu Tyr Leu Lys
        35                  40                  45

Gly Arg Lys Met Phe Val Met Leu Thr Cys Ala Phe Arg Tyr Gly His
    50                  55                  60

Asp Asp Leu Asp Val Ile Gly Leu Thr Phe Arg Lys Asp Leu Tyr Val
65                  70                  75                  80

Gln Val Gln Gln Val Val Pro Ala Glu Pro Thr Ser Pro Gln Val Pro
                85                  90                  95

Leu Thr Val Leu Gln Glu Arg Leu Leu His Lys Leu Gly Asp Asn Ala
            100                 105                 110

Tyr Pro Phe Asn Leu Gln Met Val Val Asn Leu Pro Cys Ser Val Thr
        115                 120                 125

Leu Gln Pro Gly Pro Asp Asp Thr Gly Lys Ala Cys Gly Ile Asp Phe
    130                 135                 140

Glu Val Lys Ser Phe Cys Ala Glu Asn Leu Glu Glu Lys Val Ser Lys
145                 150                 155                 160

Lys Asp Ser Val Arg Leu Val Ile Arg Lys Ile Gln Phe Ala Pro Val
                165                 170                 175

Glu Pro Gly Pro Gly Pro Trp Ala Gln Thr Val Arg Arg Phe Leu Leu
            180                 185                 190

Ser Ala Gln Pro Leu Gln Leu Gln Ala Trp Met Asp Arg Glu Val His
        195                 200                 205

Tyr His Gly Lys Pro Ile Ser Val Asn Val Ser Ile Asn Asn Ser Thr
    210                 215                 220

Asn Lys Val Ile Lys Lys Ile Lys Ile Ser Val Asp Gln Ile Thr Asp
225                 230                 235                 240

Val Val Leu Tyr Ser Leu Asp Lys Tyr Thr Lys Thr Val Phe Ile Gln
                245                 250                 255

Glu Phe Thr Glu Thr Ile Ala Ala Asn Ser Thr Phe Ser Lys Ser Phe
            260                 265                 270

Glu Val Thr Pro Leu Leu Ala Asp Asn Cys Glu Lys Gln Gly Leu Ala
        275                 280                 285

Leu Asp Gly Lys Leu Lys His Gly Asp Thr Asn Leu Ala Ser Ser Thr
    290                 295                 300

Ile Leu Arg Pro Gly Met Asp Lys Glu Leu Leu Gly Ile Leu Val Ser
305                 310                 315                 320

Tyr Lys Val Arg Val Asn Leu Met Val Ser Cys Gly Gly Ile Leu Gly
                325                 330                 335

Asp Leu Thr Ala Ser Asp Val Gly Val Glu Leu Pro Leu Ile Leu Met
            340                 345                 350

His Pro Lys Pro Ser Asn Glu Ala Ala Ser Ser Glu Asp Ile Val Ile
        355                 360                 365

Glu Glu Phe Ala Arg Gln Glu Pro Gly Gly Arg Glu Glu Ser Gln Glu
    370                 375                 380

Ala Leu Ala Ala Glu Gly Asp Glu Gly Ser
385                 390

<210>  7
<211>  1490
<212>  DNA
<213>  Sus scrofa

<220>
<221>  CDS
<222>  (129)..(1346)

<400>  7
atctggtcag aagaggctgc tgctgctacc aacggacaga acctctgtcc tgcaccctga      60

gcccatctgc gtgcaccagc ctcgaaccac ctgacgcaca tccaaggtgg gagaaactgc     120

cagatacc atg gca gcc aac gtg cag gcc agt aag tct acc cca aac cac      170
         Met Ala Ala Asn Val Gln Ala Ser Lys Ser Thr Pro Asn His
         1               5                   10

gtc att ttc aag aag acc tcc cgg gac aaa tcg gtg acc atc tac ctg      218
Val Ile Phe Lys Lys Thr Ser Arg Asp Lys Ser Val Thr Ile Tyr Leu
15                  20                  25                  30

ggg aag aga gac tat ata gac cat gtc gac caa gtg gag cct gtg gat      266
Gly Lys Arg Asp Tyr Ile Asp His Val Asp Gln Val Glu Pro Val Asp
                35                  40                  45

ggt gtc gtg ttg gtg gac cct gag ctt gtg aag gga aag aga gtg tac      314
Gly Val Val Leu Val Asp Pro Glu Leu Val Lys Gly Lys Arg Val Tyr
            50                  55                  60

gtc tct ctg acc tgc gcc ttc cgc tac ggc cag gaa gac atc gat gtg      362
Val Ser Leu Thr Cys Ala Phe Arg Tyr Gly Gln Glu Asp Ile Asp Val
        65                  70                  75

atc ggc ctg agc ttc cgc agg gac ctg tac ttc tcc cag gtc cag gtg      410
Ile Gly Leu Ser Phe Arg Arg Asp Leu Tyr Phe Ser Gln Val Gln Val
    80                  85                  90

ttc cct ccc gtg ggg gcc gcg agc caa acg aag ctg cag gag agc ctg      458
Phe Pro Pro Val Gly Ala Ala Ser Gln Thr Lys Leu Gln Glu Ser Leu
95                  100                 105                 110

atc aag aag ctg ggg ggc cac acg tac cct ttc ctg ctc acg ttt cct      506
Ile Lys Lys Leu Gly Gly His Thr Tyr Pro Phe Leu Leu Thr Phe Pro
                115                 120                 125

gac tac ctg ccc tgc tcg gtg atg ctg cag ccg gct ccg caa gac gtg      554
Asp Tyr Leu Pro Cys Ser Val Met Leu Gln Pro Ala Pro Gln Asp Val
            130                 135                 140

ggg aag tgc tgt ggg gtt gac ttc gag gtc aaa gca ttc gcc aca gac      602

Gly Lys Cys Cys Gly Val Asp Phe Glu Val Lys Ala Phe Ala Thr Asp
        145                 150                 155

agt gca gat acc gga gag gac aaa att ccc aag aag agc tct gtg cgt      650
Ser Ala Asp Thr Gly Glu Asp Lys Ile Pro Lys Lys Ser Ser Val Arg
    160                 165                 170

ttg ctg atc cgg aag gta cag cac gca ccc ctc gag atg ggt ccc cag      698
Leu Leu Ile Arg Lys Val Gln His Ala Pro Leu Glu Met Gly Pro Gln
175                 180                 185                 190

ccc cac gcg gag gcc gcc tgg caa ttc ttc atg tcc gac aag ccc ctg      746
Pro His Ala Glu Ala Ala Trp Gln Phe Phe Met Ser Asp Lys Pro Leu
                195                 200                 205

cac ctc acc gtc tcc ctc agc aaa gag ctc tat tac cac ggg gaa ccc      794
His Leu Thr Val Ser Leu Ser Lys Glu Leu Tyr Tyr His Gly Glu Pro
            210                 215                 220

att cct gtg acc gtg act gtg acc aat aac acg gag aag acc gtg aag      842
Ile Pro Val Thr Val Thr Val Thr Asn Asn Thr Glu Lys Thr Val Lys
        225                 230                 235

aag atc aaa gca cta gtg gaa caa gtg gcc aac gtg gtt ctc tac tcg      890
Lys Ile Lys Ala Leu Val Glu Gln Val Ala Asn Val Val Leu Tyr Ser
    240                 245                 250

agt gat tat tac atc aag ccg gtt gcc acg gag gaa aca cag caa aaa      938
Ser Asp Tyr Tyr Ile Lys Pro Val Ala Thr Glu Glu Thr Gln Gln Lys
255                 260                 265                 270

gtg cca cca aac agc acc ctg acc aca acg ctg acg ctg gtg ccc ttg      986
Val Pro Pro Asn Ser Thr Leu Thr Thr Thr Leu Thr Leu Val Pro Leu
                275                 280                 285

ctg gct aat aac cga gag aga agg ggc atc gct gat ggg aag atc aag      1034
Leu Ala Asn Asn Arg Glu Arg Arg Gly Ile Ala Asp Gly Lys Ile Lys
            290                 295                 300

cac gag gac acg aac ctt gcc tcc agc acc atc ata aag gaa ggc atg      1082
His Glu Asp Thr Asn Leu Ala Ser Ser Thr Ile Ile Lys Glu Gly Met
        305                 310                 315

gac aag acc atc atg ggc atc ctg gtg tct tac cag atc aag gtg cag      1130
Asp Lys Thr Ile Met Gly Ile Leu Val Ser Tyr Gln Ile Lys Val Gln
    320                 325                 330

ctc acg gtg tct ggc ctc ctg gga gag ctc acc tcc agt gaa gtg gcc      1178
Leu Thr Val Ser Gly Leu Leu Gly Glu Leu Thr Ser Ser Glu Val Ala
335                 340                 345                 350

act gag gtg ccg ttc cgc ctc atg cac ccc cag ccc gag gac cca gag      1226
Thr Glu Val Pro Phe Arg Leu Met His Pro Gln Pro Glu Asp Pro Glu
                355                 360                 365

gcc atg gac agt ttt cag gat gaa aat ttt gtt ttt gag gag ttt gcc      1274
Ala Met Asp Ser Phe Gln Asp Glu Asn Phe Val Phe Glu Glu Phe Ala
            370                 375                 380

cgc caa aat ctg aaa gat gca gga gaa ctt aag gaa gag aag aca gac      1322
Arg Gln Asn Leu Lys Asp Ala Gly Glu Leu Lys Glu Glu Lys Thr Asp
        385                 390                 395

cag gag gcg gcg gct cat gag tga agaggtcggc cccgggcacg ggctgcgggg      1376
Gln Glu Ala Ala Ala His Glu
    400                 405

tccttagcag gcgcgcccca gccgcccgca tgcagccttt agttgtgctg aaattaccag      1436

gaggtgtggg tcttgctcac agaaaattaa aaggttttgt ctcttacgct gaaa          1490

<210>  8
<211>  405
<212>  PRT
<213>  Sus scrofa

<400>  8

Met Ala Ala Asn Val Gln Ala Ser Lys Ser Thr Pro Asn His Val Ile
1               5                   10                  15

Phe Lys Lys Thr Ser Arg Asp Lys Ser Val Thr Ile Tyr Leu Gly Lys
            20                  25                  30

Arg Asp Tyr Ile Asp His Val Asp Gln Val Glu Pro Val Asp Gly Val
        35                  40                  45

Val Leu Val Asp Pro Glu Leu Val Lys Gly Lys Arg Val Tyr Val Ser
    50                  55                  60

Leu Thr Cys Ala Phe Arg Tyr Gly Gln Glu Asp Ile Asp Val Ile Gly
65                  70                  75                  80

Leu Ser Phe Arg Arg Asp Leu Tyr Phe Ser Gln Val Gln Val Phe Pro
                85                  90                  95

Pro Val Gly Ala Ala Ser Gln Thr Lys Leu Gln Glu Ser Leu Ile Lys
            100                 105                 110

Lys Leu Gly Gly His Thr Tyr Pro Phe Leu Leu Thr Phe Pro Asp Tyr
        115                 120                 125

Leu Pro Cys Ser Val Met Leu Gln Pro Ala Pro Gln Asp Val Gly Lys
    130                 135                 140

Cys Cys Gly Val Asp Phe Glu Val Lys Ala Phe Ala Thr Asp Ser Ala
145                 150                 155                 160

Asp Thr Gly Glu Asp Lys Ile Pro Lys Lys Ser Ser Val Arg Leu Leu
                165                 170                 175

Ile Arg Lys Val Gln His Ala Pro Leu Glu Met Gly Pro Gln Pro His
            180                 185                 190

Ala Glu Ala Ala Trp Gln Phe Phe Met Ser Asp Lys Pro Leu His Leu
        195                 200                 205

Thr Val Ser Leu Ser Lys Glu Leu Tyr Tyr His Gly Glu Pro Ile Pro
    210                 215                 220

Val Thr Val Thr Val Thr Asn Asn Thr Glu Lys Thr Val Lys Lys Ile

225                 230                 235                 240

Lys Ala Leu Val Glu Gln Val Ala Asn Val Val Leu Tyr Ser Ser Asp
                245                 250                 255

Tyr Tyr Ile Lys Pro Val Ala Thr Glu Glu Thr Gln Gln Lys Val Pro
            260                 265                 270

Pro Asn Ser Thr Leu Thr Thr Thr Leu Thr Leu Val Pro Leu Leu Ala
        275                 280                 285

Asn Asn Arg Glu Arg Arg Gly Ile Ala Asp Gly Lys Ile Lys His Glu
    290                 295                 300

Asp Thr Asn Leu Ala Ser Ser Thr Ile Ile Lys Glu Gly Met Asp Lys
305                 310                 315                 320

Thr Ile Met Gly Ile Leu Val Ser Tyr Gln Ile Lys Val Gln Leu Thr
                325                 330                 335

Val Ser Gly Leu Leu Gly Glu Leu Thr Ser Ser Glu Val Ala Thr Glu
            340                 345                 350

Val Pro Phe Arg Leu Met His Pro Gln Pro Glu Asp Pro Glu Ala Met
        355                 360                 365

Asp Ser Phe Gln Asp Glu Asn Phe Val Phe Glu Glu Phe Ala Arg Gln
    370                 375                 380

Asn Leu Lys Asp Ala Gly Glu Leu Lys Glu Glu Lys Thr Asp Gln Glu
385                 390                 395                 400

Ala Ala Ala His Glu
                405